# EUROPEAN PATENT APPLICATION

(11) **EP 3 109 226 A1**
(43) Date of publication of application: **28.12.2016**
(21) Application number: 15173836.6
(22) Date of filing: 25.06.2015
(51) Int. Cl.: C07C 45/41, C07C 47/565

(54) **PROCESS FOR PREPARING VANILLIN**

(71) Applicant: BASF SE, 67056 Ludwigshafen am Rhein (DE)
(72) Inventor: FENLON, Thomas, 68165 Mannheim (DE); RUEDENAUER, Stefan, 69469 Weinheim (DE); MILICIC, Rafael, 67065 Ludwigshafen (DE)
(74) Representative: Reitstötter Kinzebach

(57) **Abstract**

The present invention relates to a process for producing 4-hydroxy-3-methoxybenzaldehyde (vanillin), which comprises providing 4-hydroxy-3-methoxybenzoic acid chloride and hydrogenating the 4-hydroxy-3-methoxybenzoic acid chloride with hydrogen in the presence of a supported metal catalyst.

## Description

The present invention relates to a process for producing 4-hydroxy-3-methoxybenz-aldehyde (vanillin), which comprises providing 4-hydroxy-3-methoxybenzoic acid chloride and hydrogenating the 4-hydroxy-3-methoxybenzoic acid chloride with hydrogen in the presence of a supported metal catalyst.

### BACKGROUND OF THE INVENTION

4-Hydroxy-3-methoxybenzaldehyde (vanillin), is a synthetic aroma substance, which is widely used instead of expensive natural vanilla. Vanillin is of great commercial interest as fragrance or as flavor due to its characteristic organoleptic properties. In particular, vanillin is used as an aroma substance for foods, as a fragrance in deodorants and perfumes, and also for flavor enhancement of pharmaceuticals and vitamin preparations. Vanillin is also an intermediate in the synthesis of various medicaments, such as L-dopa, methyldopa and papaverin.

Today, the vast majority of the demanded vanillin is produced synthetically from aromatic precursors, e.g. from eugenol or guaiacol, which are available in large amounts from natural and/or petrochemical sources.

A valuable aromatic precursor and/or intermediate for the production of vanillin is 4-hydroxy-3-methoxybenzoic acid (vanillic acid). Several processes for the production of vanillin from vanillic acid are known or suggested in the state of the art.

WO96/26176 for instance describes a method for preparing aromatic carboxylic acids, in particular 4-hydroxybenzioc acid derivatives comprising at least one ether group, by carboxylating phenol ethers, in particular guaiacol and guenol, using carbon dioxide. This document further describes the potential use of the thus obtained 4-hydroxybenzioc acid derivatives as intermediates for the preparation of 4-hydroxybenzaldehydes, such as vanillin.

EP 0539274 describes a process for the transformation of acids to aldehydes using hydrogen in the presence of a bimetallic rutheniuim/tin catalyst. The reaction is performed in gas-phase at temperatures ranging from 150 to 500 °C. Vanillic acid is mentioned as a potential starting material besides many other preferred carboxylic acids.

DE4428994 describes a process for the production of aromatic aldehydes from aromatic carboxylic acids using hydrogen in the presence of a catalyst comprising zirconium dioxide and a lanthanide. The reaction is performed in gas-phase at temperatures ranging from 200 to 450 °C. Also here, vanillic acid is mentioned as a potential starting material.

WO97/17134 describes a method for preparing a bimetallic rutheniuim/tin catalyst and the use of this catalyst for the reduction of carboxylic acids or their esters or anhydrides to the corresponding aldehydes in the presence of hydrogen. The reaction is performed in gas-phase at temperatures ranging from 100 to 500 °C. Vanillic acid is mentioned as a potential starting material besides many other preferred carboxylic acids.

JP 2008222679 describes a method for the reduction of aromatic compounds having a carboxyl group, e.g. 4-hydroxy-3-methoxybenzoic acid, to the corresponding aldehydes at 100 to 300 °C using water in the subcritical state. In an specific working example 4-hydroxy-3-methoxybenzoic acid was dissolved in distilled water and heated to 250 °C for 60 minutes at a pressure of 3 MPa. The obtained aqueous solution was analyzed by high performance liquid chromatography and mass spectroscopy. No yields are given.

These known synthetic processes, which aim for the one-step production of vanillin from vanillic acid, are either performed in gas-phase or under subcritical conditions. Such harsh reaction conditions typically lead to the formation of side products. Application of these methods to the production of vanillin from vanillic acid generally give unsatisfactory yields and are thus unsuitable for the effective production of vanillin from vanillic acid, in particular for technical scale production.

Also known in the state of the art are bio-catalysed transformations of 4-hydroxy-3-methoxybenzoic acid to vanillin.

WO00/17319 for instance describes a biochemical process for the production of vanillin from a carbohydrate source, in particular glucose. The last biotransformation step of this process comprises the reduction of 4-hydroxy-3-methoxybenzoic acid to vanillin using recombinant aryl-aldehyde dehydrogenase as catalyst, which is provided by a microbe, in the presence of NADP⁺, ATP and glucose 6-phosphate dehydrogenase. The overall yield for the conversion of 4-hydroxy-3-methoxybenzoic acid to vanillin is 66 %.

The known microbe-catalized transformations of 4-hydroxy-3-methoxybenzoic acid to vanillin are generally laborious and the obtainable space-time yields are low. These proecesses are thus unsuitable for the economic production of vanillin on technical scales.

### SUMMARY OF THE INVENTION

It is therefore an object of the present invention to provide an improved process for the production of 4-hydroxy-3-methoxybenzaldehyde (vanillin) from the readily available aromatic precursor 4-hydroxy-3-methoxybenzoic acid (vanillic acid) in high yields. The process should be simple and efficient to allow an economic production of 4-hydroxy-3-methoxybenzaldehyde on technical scales.

It was surprisingly found that 4-hydroxy-3-methoxybenzaldehyde (vanillin) can be prepared in high yields from 4-hydroxy-3-methoxybenzoic acid chloride, which is readily obtainable from 4-hydroxy-3-methoxybenzoic acid, using hydrogen in the presence of a supported metal catalyst.

Therefore, the present invention relates to a process for preparing 4-hydroxy-3-methoxybenzaldehyde (vanillin), which comprises
a) providing 4-hydroxy-3-methoxybenzoic acid chloride,
b) hydrogenating the 4-hydroxy-3-methoxybenzoic acid chloride obtained in step a) with hydrogen in the presence of a supported metal catalyst.

The process is efficient and allows the production of 4-hydroxy-3-methoxybenzaldehyde in high space-time yields starting from a cheap and readily available aromatic precursor. The technical applicability of the process is simple and inexpensive. By using the process according to the present invention, 4-hydroxy-3-methoxybenzaldehyd can be provided without difficulty on industrial scales.

### DETAILED DESCRIPTION

### Step a):

According to the present invention, step a) comprises the preparation of 4-hydroxy-3-methoxybenzoic acid chloride. 4-Hydroxy-3-methoxybenzoic acid chloride can, by way of example, be obtained in a convenient way from 4-hydroxy-3-methoxybenzoic acid (vanillic acid). Vanillic acid, in turn, can be obtained in large quantities from natural sources or can be, by way of example, synthesized from eugenol or guaiacol using processes that are known to the skilled person.

In a preferred embodiment of the present process the 4-hydroxy-3-methoxybenzoic acid chloride in step a) is prepared by chlorinating 4-hydroxy-3-methoxybenzoic acid using a chlorinating agent.

Generally, all common chlorinating agents known to the skilled person to be suitable for the transformation of carboxylic acids into their corresponding acid chlorides can be used for the chlorination of 4-hydroxy-3-methoxybenzoic acid.

Preferably, the chlorinating agent used in step a) of the present process is selected from thionyl chloride, oxalyl chloride, phosphoryl chloride, phosphorous pentachloride, phosgene, diphosgene or triphosgene.

More preferably, the chlorinating agent used in step a) is selected from thionyl chloride, oxalyl chloride, phosphorous pentachloride or phosgene. In particular, the chlorinating agent used in step a) is selected from thionyl chloride and oxalyl chloride.

Typically, the chlorinating agent is used in an amount of from 1.0 to 1.5 equivalents, preferably in an amount of from 1.05 to 1.4 equivalents, in particular in an amount of from 1.1 to 1.3 equivalents, based on the total amount of 4-hydroxy-3-methoxybenzoic acid in the reaction mixture.

The chlorination reaction can be carried out in the temperature range of from 20 to 120°C, preferably in the range of from 50 to 100°C, in particular in the range of from 60 to 80°C.

The chlorination reaction is typically performed at ambient pressure or at reduced or elevated pressure. It is preferable that the chlorination reaction is carried out at ambient pressure.

The chlorination reaction can be carried out in the presence or in the absence of an organic solvent. It is preferred that the chlorination reaction is carried out in the presence of an inert organic solvent. The expression "inert organic solvent" generally means an organic solvent, which under the prevailing reaction conditions does not enter into any reactions with the starting materials or reagents participating in the reaction, or with the resultant products.

Suitable inert organic solvents include, but are not limited to the following groups:
- group S1: aliphatic and alicyclic hydrocarbons, in particular alkanes and cycloalkanes having 5 to 12 carbon atoms and mixtures of these alkanes and cycloalkanes, such as pentane, hexane, heptane, octane, ligroin, petrol ether or cyclohexane;
- group S2: halogenated aliphatic hydrocarbons, such as dichloromethane, trichloromethane, tetrachloromethane or dichloroethane, and mixtures thereof;
- group S3: aromatic hydrocarbons, such as benzene, toluene, xylenes, ethylbenzene or tetralin, and mixtures thereof;
- group S4: halogenated aromatic hydrocarbons, such as chlorobenzene, dichlorobenzene, 2-chlortoluene, 3-chlortoluene or 4-chlorotoluene, and mixtures thereof;
- group S5: aliphatic or alicyclic ethers, such as methyl-tert.-butylether, dibutyl ether, tetrahydrofurane, 1,4-dioxane or 1,2-dimethoxyethane;
as well as mixtures of the aforementioned solvents.

Preferably, the inert organic solvent used in the chlorination reaction of step a) is selected from solvents of the groups S3, S4 and S5 and mixtures thereof. More preferably, the inert organic solvent used in the chlorination reaction is selected from solvents of the groups S3, S4 and S5, having a boiling point at atmospheric pressure of at least 90 °C, preferably of at least 100 °C, and mixtures thereof. Even more preferably, the inert organic solvent used in the chlorination reaction is selected from solvents of the group S3, having a boiling point at atmospheric pressure of at least 100 °C, for example 110 °C or 140 °C, and mixtures thereof. In particular the inert organic solvent used in the chlorination reaction is selected from toluene or xylenes and mixtures thereof.

Typically, the concentration of 4-hydroxy-3-methoxybenzoic acid in the reaction mixture is in the range of from 1 to 400 g/L, preferably in the range of from 20 to 200 g/L, in particular in the range of from 50 to 150 g/L.

The chlorination reaction can take place in the absence of or in the presence of an inert gas. The expression "inert gas" generally means a gas, which under the prevailing reaction conditions does not enter into any reactions with the starting materials, reagents, or solvents participating in the reaction, or with the resultant products. It is preferable that the chlorination reaction takes place without addition of any inert gas.

After the chlorination reaction has finished, the reaction mixture may eventually contain remaining chlorination reagent, in particular, if an excess of the chlorination reagent was used. It is preferred, that after the chlorination reaction has finished, any remaining chlorination reagent, if present, is at least partially removed from the product mixture via distillation under reduced pressure. During removal of the remaining chlorination reagent, it is preferred that the temperature of the product mixture is kept below 50°C, e.g. from 5 to 45 °C. It is further preferred in this connection, that any solvent that may distill off during removal of the remaining chlorination reagent is afterwards re-added to the product mixture.

The vanillic acid chloride, prepared according to step a) of the present invention, is typically obtained in the form of a stable solution in the inert organic solvent. It is found to be advantageous for the stability of the vanillic acid chloride solution, if high concentrations of the vanillic acid chloride are avoided, since these may lead to an undesirable increase in the formation of vanillic acid dimers or oligomers. Furthermore, the solution should preferably be kept at temperatures below 50°C, e.g. from 5 to 45 °C.

In a preferred embodiment of the present invention, the 4-hydroxy-3-methoxybenzoic acid chloride in step a) is obtained in the form of a solution in the inert organic solvent, where the concentration of 4-hydroxy-3-methoxybenzoic acid chloride in the solution is in the range of from 1 to 400 g/L, preferably in the range of from 20 to 200 g/L, in particular in the range of from 50 to 150 g/L.

The chlorination reaction can be designed to take place either continuously or batchwise. The batchwise oxidation can be conducted in a reaction apparatus conventionally used for this purpose, e.g. a stirred reactor, which is optionally equipped with metering devices. The chlorination reaction may also be carried out continuously, e.g. in a tube reactor or in a cascade of at least two stirred reactors, which may be back-mixed or not.

### Step b)

Step b) of the present invention comprises the hydrogenation of 4-hydroxy-3-methoxybenzoic acid chloride obtained in step a) to 4-hydroxy-3-methoxybenzaldehyde (vanillin) using hydrogen in the presence of a supported metal catalyst.

Suitable catalysts for the hydrogenation of 4-hydroxy-3-methoxybenzoic acid chloride are for example supported metal catalysts, which comprise at least one metal of transition group VIII of the Periodic Table of the Elements, preferably palladium, osmium platinum or nickel, in particular palladium, either alone or together with at least one metal from transition group I or VII of the Periodic Table of the Elements, for example copper, zinc or rhenium.

The catalyst used in step b) of the present invention is typically deposited on a support material. Generally, any support material that is described in the state of the art for such catalysts can be used. Suitable support materials are by way of example single or mixed metal oxides, such as zirconium dioxide (ZrO₂), zinc oxide (ZnO), magnesium oxide (MgO), titanium dioxide (TiO₂), aluminium oxide, TiO₂-Al₂O₃ or ZrO₂-Al₂O₃, zeolites (aluminosilicates), hydrotalcite, silicium carbide (SiC), tungsten carbide (WC), silicium dioxide (SiO₂), charcoal, activated charcoal, carbon, sulfated carbon, diatomite, clay, barium sulfate, calcium carbonate or else a combination thereof.

In a preferred embodiment of the present invention the supported metal catalysts that are used in step b) of the present process contain palladium as the catalytically active metal.

The palladium containing supported metal catalysts may comprise exclusively palladium as the catalytically active metal or mixtures of palladium with other catalytically active metals different from palladium, as described above. The catalytically active metals different from palladium may also be present in trace amounts as dopants.

Preferably, the supported palladium containing catalyst used in step b) of the present invention comprises no other metals than palladium as the catalytically active metal.

If the supported metal catalyst used in step b) of the present invention contains a highly catalytically active metal, such as palladium, it is often desirable to reduce the catalytic activity of supported metal catalyst in order to prevent subsequent reduction reaction of the obtained 4-hydroxy-3-methoxybenzaldehyde and thus, to increases the selectivity of the hydrogenation reaction.

The catalytic activity of the supported palladium containing catalysts used in step b) of the present invention can for example be lowered by the addition of a regulator (catalyst poison). Alternatively or additionally, the activity of the supported palladium containing catalyst can be reduced by immobilizing the catalyst on a support material having a low surface area, such as barium sulfate.

If the supported metal containing catalysts used in step b) of the present process contains palladium as the predominant catalytically active metal, it is preferable that the catalyst comprises a regulator (catalyst poison) and/or that the catalyst is immobilized on a support material having a low surface area, such as barium sulfate.

Several suitable regulators are described in the art (see for example Mosettig and Mozingo, "The Rosenmund Reduction of Acid Chlorides to Aldehydes", Organic Reactions, 1948, Vol. 4, pp. 362 to 377 or US 3,517,066). They are typically selected, from quinoline, sodium acetate, sulfur or sulfur-containing compounds, such as for example sulfur in quinoline (quinoline-S), thioquinanthrene, 2-mercaptobenzothiazole, phenylisothiocyanate or thiourea.

Examples of preferred supported metal catalysts that can advantageously be used in step b) of the present invention are Pd on barium sulfate or Pd on charcoal, optionally poisoned with sulfur or quinoline-S.

A particularly preferred supported metal catalyst that is used in step b) of the present process is palladium on barium sulfate.

Typically, the metal content of the supported metal catalyst is in the range of from 0.1 to 30% by weight, preferably in the range of from 0.5 to 20% by weight and in particular of from 1 to 15% by weight.

The supported metal catalyst used in step b) of the process generally possess a specific BET surface area, determined by nitrogen adsorption at 77 K in accordance with DIN ISO 9277 2003-5, in the range of from 10 to 500 m²/g, preferably in the range of from 20 to 300 m²/g.

The supported metal catalyst may be present in the form of a powder, irregular granulates or shaped bodies, for example in the form of spheres, rings, cylinders, cubes, cuboids or other geometric bodies. The shape of the supported metal catalyst is typically determined by the shape of the support. Alternatively, the support can be subjected to a shaping process before or after the application of the catalytically active metal(s). The catalysts may, for example, be used in the form of pressed cylinders, tablets, pellets, wagonwheels, rings, stars or extrudates, such as solid extrudates, polylobal extrudates, hollow extrudates and honeycombs or other geometric bodies.

Typically, the content of the catalytically active metal in the reaction mixture of step b) is in the range of from 0.1 to 10% by weight, preferably in the range of from 0.2 to 5% by weight, in particular in the range of from 0.4 to 1.5% by weight, based on the total amount of 4-hydroxy-3-methoxybenzoic acid chloride present in the reaction mixture. The hydrogenation reaction can be carried out in the presence or in the absence of an organic solvent. It is preferred that the hydrogenation reaction is carried out in the presence of an inert organic solvent as defined above for step a) of the present process. Preferably, the inert organic solvent used in the hydrogenation reaction is selected from solvents of the groups S3 and S4 and mixtures thereof. More preferably, the inert organic solvent used in the hydrogenation reaction is selected from solvents of the group S3 and mixtures thereof, in particular from toluene or xylenes and mixtures thereof.

In a preferred embodiment of the present invention, the 4-hydroxy-3-methoxybenzoic acid chloride is reacted in the form of a solution in the inert organic solvent, where the concentration of 4-hydroxy-3-methoxybenzoic acid chloride in the solution is in the range of from 1 to 400 g/L, preferably in the range of from 20 to 200 g/L, in particular in the range of from 50 to 150 g/L.

Preferably, the 4-hydroxy-3-methoxybenzoic acid chloride obtained in step a) is directly used as starting material in the hydrogenation reaction of step b) without further work-up or purification.

The hydrogenation of acid chlorides to the corresponding aldehydes with hydrogen in the presence of a supported palladium containing catalyst can take place by analogy with known processes for hydrogenating organic compounds which have hydrogenatable groups. Suitable processes are for example described in Rosemund et al., Ber. Dtsch. Chem. Ges., 1918, Vol. 51, pp. 591, Mosettig and Mozingo, "The Rosenmund Reduction of Acid Chlorides to Aldehydes", Organic Reactions, 1948, Vol. 4, pp. 362 to 377 or in US 3,517,066. To this end, the 4-hydroxy-3-methoxybenzoic acid chloride in the form of liquid phase is brought into contact with the catalyst in the presence of hydrogen. The liquid phase can by way of example be passed over a fluidized bed of catalyst (fluidized bed method) or can be passed over a fixed bed of catalyst (fixed bed method).

Suitable apparatuses for conducting fluidized-bed-catalyst hydrogenation and fixed-bed-catalyst hydrogenation are known in the prior art, e.g. from Ullmanns Enzyklopädie der Technischen Chemie [Ullmann's Encyclopedia of Industrial Chemistry], 4th edition, volume 13, pp. 135 ff., and also from P. N. Rylander, "Hydrogenation and Dehydrogenation" in Ullmann's Encyclopedia of Industrial Chemistry, 5th edn. on CD-ROM.

The catalytic hydrogenation is generally carried out at a hydrogen pressure in the range of from 0.1 to 50 bar, preferably in the range of from 0.3 to 20 bar, more preferably in the range of from 0.5 to 10 bar, particularly in the range of from 0.7 to 5 bar.

In a preferred embodiment of the present invention, the hydrogen is introduced to the hydrogenation reaction in step b) in the form of a hydrogen stream.

The hydrogen stream can be passed into the gas space of the reaction system or into the liquid reaction mixture. The introduction of the hydrogen stream into the reaction system preferably takes place in a manner that creates a large area for interchange between the liquid reaction mixture and the inert gas. The introduction of the hydrogen stream into the reaction system during the reaction has a stripping effect and enables the removal of the hydrochloric acid or hydrogen chloride that is liberated during the reaction.

Additionally or alternatively, organic bases, such as alkaline salts of organic acids, e.g. sodium acetate, which fix the hydrochloric acid or hydrogen chloride liberated, can be added to the catalytic hydrogenation reaction of step b). Tertiary amides, such as N,N-dimethylacetamide, are, by way of example, also suitable for this purpose (see for example US 3,996,292 A).

It is preferred, that the hydrogen stream is introduced into the reaction mixture below the liquid surface in such a way that it bubbles through the reaction mixture. The pressure of the hydrogen stream must be sufficiently high to overcome the hydrostatic pressure of the reaction mixture above the inert gas feed.

The hydrogen stream can be fed into the system by way of any desired suitable apparatuses. Among these are by way of example nozzles for gas-supply lances. The nozzles can be on the base of the reactor or in the vicinity of the base. There can also be a plurality of nozzles e.g. arranged in the form of a ring.

It is preferable that the reaction mixture is mixed in order to bring about an interchange of reaction mixture in the reactor region below the feed of the hydrogen stream with reaction mixture in the reactor region above the feed of the hydrogen stream. By way of example, stirrers or a circulating pump are suitable for the mixing process. In one specific variant, what is known as a gas-introducing stirrer is used for the introduction of the hydrogen stream and for the mixing of the reaction mixture.

The introduction of the hydrogen stream into the reaction system can be performed at the start of the reaction process or during the whole course of the hydrogenation reaction. Preferably, the hydrogen stream is introduced into the reaction system at the start of the hydrogenation reaction as soon as the catalyst has been added to the reaction mixture.

The introduction of the hydrogen stream into the reaction system is typically performed for several minutes to a few hours, e.g. 30 minutes to 1.5 hours, until the majority of the liberated hydrochloric acid or hydrogen chloride is removed. During this procedure, the temperature of the reaction mixture is kept in the range from 20 to 80 °C, preferably in the range from 25 to 60°C, in particular in the range from 30 to 50 °C.

After the introduction of the hydrogen stream, the hydrogen reaction may be run under hydrogen pressure for further 1 to 24 hours, e.g. for further 5 to 15 hours, and at a temperature in the range form 30 to 120 °C, preferably in the range from 40 to 100°C, in particular in the range from 50 to 80 °C, until completion of the hydrogenation reaction.

As mentioned above, step a) and/or step b) of the process according to the present invention can be carried out in the absence or in the presence of an organic solvent.

In a preferred embodiment of the present process, step a) is carried out in the presence of an organic solvent, which is inert under the reaction conditions (inert organic solvent), as defined above.

In another preferred embodiment of the present process, step b) is carried out in the presence of an organic solvent, which is inert under the reaction conditions (inert organic solvent), as defined above.

In a more preferred embodiment of the present process, both steps a) and b) are carried out in the presence of an organic solvent, which is inert under the reaction conditions (inert organic solvent), as defined above. Regarding suitable and preferred inert organic solvents, reference is made to the definitions given above.

In an even more preferred embodiment of the present process, both steps a) and b) are carried out in the presence of an inert organic solvent selected from solvents of the group S3, having a boiling point at atmospheric pressure of at least 100 °C, for example 110 °C or 140 °C, and mixtures thereof, as defined above.

In particular, both steps a) and b) of the process are carried out in the presence of toluene or xylenes or mixtures thereof.

Step a) and step b) of the process can be carried out in different organic solvents or solvent mixtures or in the same organic solvent or solvent mixture.

Preferably, steps a) and b) of the process are carried out in the same organic solvent or solvent mixture.

After completion of the hydrogenation reaction, the inert organic solved is typically distilled off under reduced pressure. Generally, no further work-up or purification of the thus obtained 4-hydroxy-3-methoxybenzaldehyde is necessary. If the hydrogenation reaction is performed in fluidized bed method or in suspension, the catalyst is typically removed by filtration before the inert organic solvent is distilled off under reduced pressure.

The 4-hydroxy-3-methoxybenzaldehyde can typically be obtained in high purity, e.g. in a purity of at least 90%.

However, if necessary, the purity of the 4-hydroxy-3-methoxybenzaldehyde obtained in step b) of the present invention can be further increased by adding an additional purification step, preferably a distillation step.

Suitable distillation devices for the purification of 4-hydroxy-3-methoxybenzaldehyde are for example distillation columns, such as tray columns optionally equipped with bubble cap trays, sieve plates, sieve trays, packages or filler materials, or spinning band columns, such as thin film evaporators, falling film evaporators, forced circulation evaporators, Sambay evaporators, etc. and combinations thereof.

The hydrogenation can be designed to take place either continuously or batchwise. The batchwise hydrogenation can be conducted in a reaction apparatus conventionally used for this purpose, e.g. a stirred reactor, which is optionally equipped with metering devices.

In yet another embodiment of the present process, step b) or both steps a) and b) are performed in a continuous manner, e.g. in a tube reactor or in a cascade of at least two stirred reactors, which may be back-mixed or not.

### EXAMPLES

### Example I.1: Synthesis of vanilloyl chloride from vanillic acid

10.0 g vanillic acid (57.7 mmol) in 100 ml toluene was heated to 70°C and 9.01 g (75 mmol) thionyl chloride was slowly added. After stirring for 2 h at 70°C, the remaining thionyl chloride was removed under reduced pressure (40°C, 70 mbar). Any toluene removed during the removal of the thionyl chloride was re-added to the product mixture. The obtained solution was immediately used for the next step (Example 1.2).

### Example I.2: Synthesis of vanillin from vanilloyl chloride

To the vanilloyl chloride solution in toluene obtained from Example I.1 2.5 g hydrogenation catalyst (5% Pd on BaSO₄) was added and heated to 40°C while a stream of hydrogen was passed through the suspension. The suspension was stirred for 10 h under hydrogen atmosphere at 70°C, after which the hydrogen was removed and replaced with N₂. The catalyst was removed via filtration, and the toluene was removed under reduced pressure to obtain vanillin in 65% yield after two steps (overall yield for Example I.1 and I.2).

## Claims

1. A process for preparing 4-hydroxy-3-methoxybenzaldehyde (vanillin), which comprises
a) providing 4-hydroxy-3-methoxybenzoic acid chloride,
b) hydrogenating the 4-hydroxy-3-methoxybenzoic acid chloride obtained in step a) with hydrogen in the presence of a supported metal catalyst.

2. The process of claim 1, where the 4-hydroxy-3-methoxybenzoic acid chloride in step a) is prepared by chlorinating 4-hydroxy-3-methoxybenzoic acid using a chlorinating agent.

3. The process of claim 2, where the chlorinating agent used in step a) is selected from thionyl chloride, oxalyl chloride, phosphoryl chloride, phosphorous pentachloride, phosphorous trichloride, cyanuric chloride, phosgene or triphosgene.

4. The process of claims 2, where the chlorinating agent used in step a) is selected from thionyl chloride or oxalyl chloride.

5. The process of claims 2 to 4, where the chlorinating agent is used in an amount of from 1.0 to 1.5 equivalents, based on the total amount of 4-hydroxy-3-methoxybenzoic acid in the reaction mixture.

6. The process of any of the preceding claims, where the 4-hydroxy-3-methoxybenzoic acid chloride in step a) is obtained in the form of a solution in an inert organic solvent, where the concentration of 4-hydroxy-3-methoxybenzoic acid chloride in the solution is in the range of from 1 to 400 g/L.

7. The process of claim 6, where the 4-hydroxy-3-methoxybenzoic acid chloride solution obtained in step a) is directly applied in step b).

8. The process of any of the preceding claims, where the supported metal catalyst used in step b) contains palladium.

9. The process of any of the preceding claims, where the supported metal catalyst used in step b) is selected from palladium on BaSO₄ or palladium on charcoal.

10. The process of any of the preceding claims, where the content of the catalytically active metal in the reaction mixture of step b) is in the range of from 0.1 to 10 % by weight, based on the total amount of 4-hydroxy-3-methoxybenzoic acid chloride present in the reaction mixture.

11. The process of any of the preceding claims, where the hydrogen is introduced to the hydrogenation reaction in step b) in the form of a hydrogen stream.

12. The process of claim 1, where step a) is performed in an inert organic solvent.

13. The process of claim 1, where step b) is performed in an inert organic solvent.

14. The process of claim 13, where in step b) the 4-hydroxy-3-methoxybenzoic acid chloride is reacted in the form of a solution in the inert organic solvent, where the concentration of 4-hydroxy-3-methoxybenzoic acid chloride in the solution is in the range of from 1 to 400 g/L.

15. The process of claims 12 to 14, where the inert organic solvent is selected from aromatic hydrocarbons.
